# EUROPEAN PATENT SPECIFICATION

(11) **EP 1 999 110 B1**
(45) Date of publication and mention of the grant of the patent: **16.09.2009**
(21) Application number: 07723445.8
(22) Date of filing: 21.03.2007
(51) Int. Cl.: C07D 213/24, C07D 471/04

(54) **PROCESS FOR PREPARING l-HALO-2,7-NAPHTHYRIDINYL DERIVATIVES**
VERFAHREN ZUR HERSTELLUNG VON 1-HALOGEN-2,7-NAPHTHYRIDINYLDERIVATEN
PROCEDE DE PREPARATION DE DERIVES 1-HALOGENO-2,7-NAPHTYRIDINYLE

(30) Priority: 22.03.2006 GB 0605766
(43) Date of publication of application: 10.12.2008
(73) Proprietor: UCB Pharma, S.A., 1070 Brussels (BE)
(72) Inventor: TYRRELL, Nicholas, David, Berkshire SL1 3WE (GB); TREMAYNE, Neil, Cambridge CB1 3LA (GB); EVANS, Graham, Robert, Newmarket, Suffolk CB8 OAD (GB)
(74) Representative: Hassa, Jürgen
(86) International application number: PCT/EP2007/002485
(87) International publication number: WO 2007/107345

(56) References cited:
- EP-A- 1 348 711
- WO-A-20/04007494
- US-A- 4 859 671

## Description

The present invention is directed towards a process for preparing 1-halo-2,7-naphthyridinyl derivatives and to a class of enamine reagents that can be used as reactants in the process. 1-Halo-2,7-naphthyridinyl derivatives are useful as intermediates in the preparation of pharmacologically active compounds. For example, International Patent Application WO 02/068393 discloses a class of phenylalanine enamide derivatives, including 2,7-naphthyridin-1-ylamine phenylalanine derivatives as potent inhibitors of α4 integrins. 1-Halo-2,7-naphthyridines are useful as intermediates in the preparation of such phenylalanine enamide derivatives.

Ikekawa, N. (Chem. Pharm, Bull, 1958, 6, 269-72) first reported the synthesis of 1-chloro-2,7-naphthyridine (CAS No. 69042-30-4) *viα* 2,7-naphthyridin-1-ol starting from 4-methyl nicotinic acid. The overall synthesis was low yielding and unsuitable for large-scale manufacture.

Subsequently Baldwin, J.J. (J. Org. Chem; 1978, 43 (25), 4878-80) reported an improved synthesis of 1-hydroxy-2,7-naphthyridine starting from 3-cyano-4-methyl pyridine (CAS No. 5444-01-9).

In Baldwin's procedure 3-cyano-4-methyl pyridine is converted into *N,N-*dimethyl-2-(3-cyano-4-pyridyl)ethenamine (trans geometry, CAS No. 67988-51-6) *via* the action of *N*,*N-*dimethyl formamide dimethyl acetal. However the reaction requires high temperatures (150°C) and long reaction times (16 hours). Furthermore the product requires purification by distillation, using high temperature and low vacuum, 150-160°C, 0.2min Hg, (US-4176183) with an overall yield that is only moderate (63%). Because of the harsh reaction conditions and the requirement for purification by distillation the procedure is inconvenient for large-scale manufacture.

In Baldwin's procedure *N,N-*dimethyl-2-(3-cyano-4-pyridyl)ethenamine (CAS No. 67988-51-6) is converted into 2,7-naphthyridin-1-ol (CAS No. 67988-50-5) by the action of hydrogen bromide in acetic acid. Whereas the reaction itself is quite efficient the isolation of the product is difficult. The product has to be isolated from aqueous media by continuous extraction because the product has significant water solubility. After continuous extraction the product requires further purification by flash chromatography to give only a moderate yield.

2,7-Naphthyridin-1-ol (CAS No. 67988-50-5) can be converted, for example, to 1-chloro-2,7-naphthyridine (CAS No. 69042-30-4) by heating the naphthyridone in phosphorus oxychloride in a sealed vessel at high temperature, for an extended period of time (cf. US 4,859,671), but this gives rise to only a moderate yield of product. In our hands the reaction, in particular on large scale, is capricious especially when run at high concentration as the reaction mixture tends to agglomerate giving rise to decomposition, lower yields and lower purity product.

WO 02/068393 also discloses a method for the preparation of 1-halo-2,7-naphthyridine derivatives using *N,N-*dimethyl-2-(3-cyano-4-pyridyl)ethenamine.

WO 04/007494 discloses the preparation of 1-chloro-2,7-naphthyridine wherein 3-cyano-4-methylpyridine is reacted with dimethylformamide dimethyl acetal or dimethylformamide diethyl acetal in DMF to produce 3-cyano-4-(N,N'-dimethylamino)ethylen-1-yl)pyridine. The said 3-cyano-4-(N,N'-dimethylamino)ethylen-1-yl)pyridine is then reacted with HCl or HBr in acetic acid to produce 1-hydroxy-2,7-naphthyridine wich is further converted into 1-chloro-2,7-naphthyridine with phosphorous oxychloride.

We have now developed a novel process for the synthesis of 1-halo-2,7-naphthyridine derivatives, via a cyclised enamine, which is particularly amenable to large scale synthesis. The process is convenient to operate and advantageously provides clean, high yielding crystalline intermediates, which do not require long reaction periods for preparation.

The present invention accordingly provides a process for the preparation of a compound of formula (I): 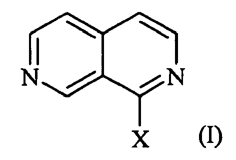 wherein
X is Cl or Br;
which process comprises the following steps:
(i) reaction of a 3-cyano-4-methylpyridine derivative of formula (A): 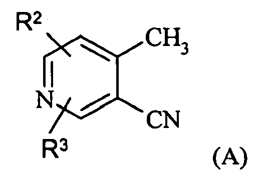 with a compound of formula (II): 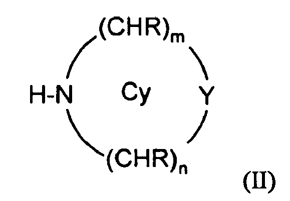 in the presence of an *N,N-*dimethylformamide diC₁₋₆alkylacetal;
   to give an enamine derivative of formula (III): 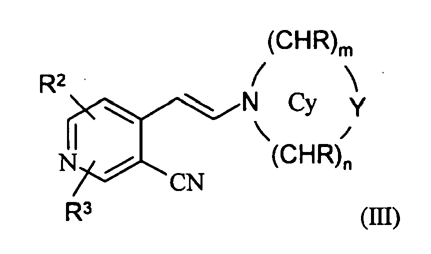 wherein R² and R³ independently represent hydrogen, Cl or Br; and Cy represents a N-linked 4 to 6 membered heterocyclic ring in which m is 1 or 2; n is 1 or 2; Y represents - CHR-, -NR¹-, -O- or -S(O)ₓ-; x is zero, 1 or 2; each R independently represents hydrogen or C₁₋₆alkyl; and R¹ represents hydrogen or C₁₋₆alkyl;
(ii) cyclisation of the enamine of formula (III), followed, as necessary, by removal of all C1 or Br atoms, to obtain the compound of formula (IV): 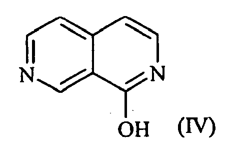
(iii) reaction of the compound of formula (IV) with a halogenating agent, to obtain a compound of formula (I).

In one embodiment X in the compound of formula (I) is a Cl atom. In another embodiment X in the compound of formula (I) is a Br atom. X in the compound of formula (I) is preferably Cl.

Suitable examples of the compound of formula (A) include 3-cyano-4-methylpyridine, 2-chloro-3-cyano-4-methylpyridine, 5-chloro-3-cyano-4-methylpyridine, 6-chloro-3-cyano-4-methylpyridine, 3-cyano-2,5-dichloro-4-methylpyridine, 3-cyano-2,6-dichloro-4-methylpyridine, 3-cyano-5,6-dichloro-4-methylpyridine, 2-bromo-3-cyano-4-methylpyridine, 5-bromo-3-cyano-4-methylpyridine, 6-bromo-3-cyano-4-methylpyridine, 3-cyano-2,5-dibromo-4-methylpyridine, 3-cyano-2,6-dibromo-4-methylpyridine, 3-cyano-5,6-dibromo-4-methylpyridine, 2-bromo-5-chloro-3-cyano-4-methylpyridine, 2-bromo-6-chloro-3-cyano-4-methylpyridine, 5-bromo-2-chloro-3-cyano-4-methylpyridine and 6-bromo-2-chloro-3-cyano-4-methylpyridine. Particular examples of the compound of formula (A) include 3-cyano-4-methylpyridine and 3-cyano-2,6-dichloro-4-methylpyridine.

In one particular embodiment R² and R³ is each hydrogen, such that the derivative of formula (A) is 3-cyano-4-methylpyridine and the compound of formula (III) is:

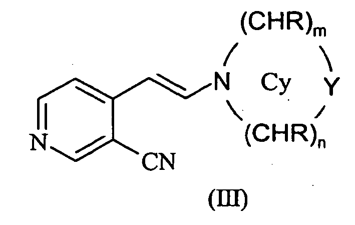

In another embodiment R² and R³ is each Cl. In a further embodiment R² is hydrogen and R³ is Cl. In yet another embodiment R² and R³ is each Br. In another embodiment R² is hydrogen and R³ is Br. In a further embodiment R² is Cl and R³ is Br.

In another particular embodiment the compound of formula (A) is 3-cyano-2,6-dichloro-4-methylpyridine.

In one embodiment, m is 1. In another embodiment, m is 2.

In one embodiment, n is 1. In another embodiment, n is 2.

Suitably, m is 1 and n is 2.

Suitably, R represents hydrogen or methyl. In one embodiment, R is hydrogen. In another embodiment, R is C₁₋₆ alkyl, especially methyl.

Suitably, R¹ represents hydrogen or methyl. In one embodiment, R¹ is hydrogen. In another embodiment, R¹ is C₁₋₆ alkyl, especially methyl.

In one embodiment, x is zero. In another embodiment, x is 1. In a further embodiment, x is 2.

In a particular embodiment, Y represents -CHR-, especially -CH₂-.

Suitable examples for the group Cy include azetidin-1-yl, pyrrolidin-1-yl, 2-methylpyrrolidin-1-yl, piperidin-1-yl, 2-methylpiperidin-1-yl, 3-methylpiperidin-1-yl, 4-methylpiperidin-1-yl, 3,5-dimethylpiperidin-1-yl, piperazin-1-yl, 4-methylpiperazin-1-yl, morpholin-4-yl, 2,6-dimethylmorpholin-4-yl, thiomorpholin-4-yl and 1,1-dioxothiomorpholin-4-yl. Particular examples of Cy include pyrrolidin-1-yl, 2-methylpyrrolidin-1-yl, piperidin-1-yl, 2-methylpiperidin-1-yl, 3-methylpiperidin-1-yl, 4-methylpiperidin-1-yl, 3,5-dimethylpiperidin-1-yl and 2,6-dimethylmorpholin-4-yl. In one embodiment of the invention Cy represents pyrrolidin-1-yl.

Representative examples of the compounds of formula (III) include 4-[2-(pyrrolidin-1-yl)vinyl]nicotinonitrile and 2,6-dichloro-4-[2-(pyrrolidin-1-yl)vinyl] nicotinonitrile.

A particular example of compound (III) is 4-[2-(pyrrolidin-1-yl)vinyl]nicotinonitrile.

The compounds of formula (III) are novel and represent another aspect of the invention.

The compounds of formula (III) may exist as either the entgegen (E) or zusammen (Z) isomer or as a mixture thereof. The invention is intended to encompass the individual isomers and mixtures thereof in any proportion.

The compound of formula (IV) may exist as tautomers and the invention is intended to encompass individual tautomers and mixtures thereof in any proportion.

The presence of certain substituents in the compounds of formulae (I), (II), (III) or (IV) may enable salts of the compounds to be used. Suitable salts include acid addition salts such as hydrochloride or hydrobromide. Compounds of formulae (I), (II), (III) or (IV) and their salts may be in the form of a solvate, such as hydrate or alcoholate, and all such solvates are included within the scope of the present invention.

Step (i) of the process described hereinabove involves the conversion of a 3-cyano-4-methylpyridine derivative of formula (A) into an enamine derivative of formula (III). The enamine of formula (III) is beneficially prepared using relatively mild reaction conditions of a short duration. Advantageously; the resultant product conveniently crystallises directly from the reaction medium in high yield and purity, thus avoiding the need for solvent extraction and further purification.

The reaction can be performed in two stages. Typically in the first stage a compound of formula (II), such as pyrrolidine, is condensed with an *N,N-*dimethylformamide diC₁₋₆alkylacetal, such as *N,N*-dimethylformamide dimethylacetal, at an elevated temperature, such as reflux, for a time of about 0.5 to 5 hours, usually 1.0 to 1.5 hours. Following distillation of the residual volatiles the resulting condensed mixture may be used in the next stage of the reaction as is. A 3-cyano-4-methylpyridine derivative of formula (A) is usually slurried in a suitable solvent, preferably a lower alcohol such as isopropyl alcohol, followed by addition of the condensation product of stage one. The reaction mixture is suitably stirred at ambient temperature or heated at an elevated temperature, e.g. reflux, for a time of about 0.5 to 6 hours, usually 1.5 to 2.0 hours. Further condensation product may be added in order for the reaction to continue to completion. Following solvent removal the resultant crystalline slurry may be filtered. When the compound of formula (A) is 3-cyano-4-methylpyridine the resulting enamine of formula (III) has been found to be obtainable in a typical yield of 87%.

Step (ii) of the process involves cyclisation of the enamine of formula (III), followed, as necessary, by removal of all Cl or Br atoms, to obtain 1-hydroxy-2,7-naphthyridine of formula (IV).

The reaction may conveniently be performed using HBr, HCl or H₃PO₄, usually HBr or HCl, typically aqueous HBr or HCl, optionally in the presence of trifluoroacetic acid. The reaction may be performed in a suitable solvent, typically an acidic solvent such as acetic acid. To illustrate, an examine of formula (III) may be stirred in acetic acid, usually at 0-25°C, typically 10-15°C, followed by addition of trifluoroacetic acid at such a rate so as to maintain the reaction temperature. The resulting mixture may be added to a mixture of water and HBr or HCl, typically HBr in acetic acid, maintaining the reaction temperature at 25-60°C, typically at 40-50°C. Once the reaction has reached completion solvent may be removed under vacuum distillation, typically azeotroping with toluene. Further solvent, typically a lower alcohol, e.g. ethanol, is suitably added from which, after further concentration, the product precipitates as the HBr or HCl salt.

In an alternative approach, concentrated hydrochloric acid can be used to cyclise the enamine. To illustrate, an enamine of formula (III) may be added to hydrochloric acid, usually at 0-25°C. The resulting mixture is heated to 60-100°C, typically at 80°C. Once the reaction has reached completion solvent may be removed under vacuum distillation. Further solvent, typically an alcohol, e.g. n-propanol or propan-2-ol, is suitably added from which, after further concentration, the product precipitates as the HCl salt hydrate.

Alternatively once the reaction has reached completion the reaction may be cooled to room temperature and the product collected by filtration.

When 3-cyano-4-methylpyridine is used in step (i) of the process the resulting compound of formula (IV) has been found to be obtainable in a typical yield of 96%. Isolation as the HBr or HCl salt advantageously removes the need for further solvent extraction and purification.

When the enamine derivative of formula (III) contains Cl or Br atoms, these are all removed after cyclisation. Typically the cyclised intermediate is treated with hydrogen gas in the presence of a suitable catalyst, such as palladium on carbon in the presence of sodium acetate, in a suitable solvent, such as a lower alcohol, for example methanol. The resulting compound of formula (IV) has been found to be obtainable in a typical yield of 64%.

Step (iii) of the process involves conversion of the hydroxy derivative of formula (IV) into the halo derivative of formula (I). Typically the hydroxy derivative is used as either the HBr or HCl salt. Advantageously use of the salt in the halogenation reaction results in a higher yielding and cleaner product than that obtained using the corresponding free base. 2,7-Naphthyridin-1-ol hydrobromide is novel and forms a further aspect of the invention.

Generally the salt of 2,7-naphthyridin-1-ol is reacted with a suitable halogenating agent, e.g. a chlorinating agent such as phosphorus oxychloride (POCl₃) or a brominating agent such as phosphorus oxybromide (POBr₃). When the HBr salt is reacted with POCl₃ the resulting product is generally a mixture of the 1-Cl- and 1-Br-2,7-naphthyridines, typically in a ratio of approximately 9:1 respectively, which can be used for further elaboration as is.

A catalytic amount of *N*,*N*-dimethylformamide (DMF) may also be employed in the reaction, beneficially resulting in a faster, cleaner and higher yielding reaction that can be performed at higher concentration.

To illustrate, POCl₃ is typically added to the HBr salt of 2,7-naphthyridin-1-ol, followed by dropwise addition of DMF. The reaction is suitably heated, usually to reflux for 1 to 5 hours, typically 3 hours. The reaction is suitably quenched by addition to a cooled mixture, typically at -10°C, of a basic aqueous solution, e.g. ammonia in water, and a suitable organic solvent such as ethyl acetate, maintaining the temperature at less than 20°C, preferably less than 15°C. After extracting the aqueous layer with further organic solvent, e.g. ethyl acetate, the organic layer may be washed with water, dried and evaporated using standard techniques. The resulting compound of formula (I) has been found to be obtainable in a typical yield of 85-96%.

Indeed, it is possible to use either the 2,7-naphthyridin-1-ol HBr or HCl salt, preferably the hydrochloride salt hydrate, in the halogenation reaction, using preferably POCl₃.

Most particularly use of the hydrate of the HBr or HCl salt in the halogenation reaction, typically the hydrate of the HCl salt in the chlorination reaction, advantageously results in a solution at the end of the reaction, compared to the use of the anhydrous HBr or HCl salt which results in a thick solid. The resulting solution is easier to quench at completion of the reaction and is especially amenable for use in large scale synthesis.

As used herein hydrate refers to crystalline forms of an organic substance in which the solvent in the crystal lattice is water. The skilled person will appreciate that hydrates can be formed by prolonged exposure to air of the corresponding anhydrous salt.

The crystalline hydrate of the HBr and HCl salts of 2,7-naphthyridin-1-ol, for example are characterised hereinafter, in particular 2,7-naphthyridin-1-ol hydrochloride hydrate.

To illustrate, POCl₃ is typically added to 2,7-naphthyridin-1-ol hydrochloride hydrate. The reaction is suitably heated, usually to reflux for 1 to 5 hours, typically 3 hours. The reaction mixture is then diluted with dichloromethane. The reaction is suitably quenched by addition to a cooled mixture of a basic aqueous solution, e.g. ammonia in water, and a suitable organic solvent such as dichloromethane, ideally maintaining the temperature at less than 20°C, preferably less than 15°C. After extracting the aqueous layer, the organic layer may be washed with water, dried and evaporated using standard techniques. The water washed organic layer may be partially distilled and then a further solvent, such as acetonitrile, may be added, followed by further partial distillation. This process may then be repeated. Water may then be added to the resulting residue, ideally maintaining the internal temperature above 50°C, followed by gradual cooling to preferably 5°C. After standing the resulting solid may be collected by filtration and typically washed with water and dried.

The resulting compound of formula (I) has been found to be obtainable in a typical yield of 85-96%.

In an alternative approach, the halogenating agent can be generated from a mixture of tetra-*n*-butylammonium chloride and phosphorus pentoxide.

The direct conversion of the enamine (III) to compound (I) where X is Br can also be achieved. Thus, in another aspect, the present invention provides a process for the preparation of a compound of formula (I) as depicted above wherein X is Br, which comprises reacting a compound of formula (III) as defined above with HBr. Here, a solution of the enamine (III) is conveniently prepared and to this cooled (e.g. -5°C) solution is suitably added gaseous hydrogen bromide. A solvent such as acetic acid may be used. Alternatively a solvent such as dichloromethane may be employed. When the reaction is complete the mixture is typically poured onto saturated NaHCO₃. The layers are then separated and the organic layer dried and evaporated giving the crude bromide (I).

In a similar manner the direct conversion of the enamine (III) to compound (I) where X is Cl can also be achieved. Thus, in another aspect, the present invention provides a process for the preparation of a compound of formula (I) as depicted above wherein X is Cl, which comprises reacting a compound of formula (III) as defined above with HCl. Typically a solution of the enamine (III) is stirred in a suitable solvent, such as acetic acid, followed by addition of HCl in, for example, acetic acid.

The following non-limiting Examples are intended to illustrate the present invention. All temperatures are in °C. The following abbreviations are used:
- EtOAc -: ethyl acetate;
- MeOH -: methanol;
- AcOH -: acetic acid;
- Ar -: aryl;
- iPr -: isopropyl;
- SG -: specific gravity;
- DMF -: N,N-dimethylformamide
- IPA -: isopropyl alcohol
- DCM -: dichloromethane;
- EtOH -: ethanol;
- DMSO -: dimethylsulphoxide;
- Me -: methyl;
- MeCN -: acetonitrile;

The IUPAC names of the compounds mentioned in the Examples were generated with ACD version 6.00.

¹H NMR spectra are recorded on a Bruker AV-300 or DRX-400 spectrometer operating at 300.13 MHz or 400.13 MHz for protons, and running the Bruker XWINNMR software package. Spectra were acquired at room temperature unless otherwise stated. Chemical shifts are given in ppm referenced to internal TMS or to the residual solvent signal.

| | | |
|---|---|---|
| **HPLC system 1** | Column Phenomenex Luna 5µ C-18 (2), 150 x 4.6 mm | |
| **Mobile phase** | Component A: 20mM pH7 KH₂PO₄ buffer; | |
| | Component B: 90% MeCN/10% HPLC water; | |
| | Flow rate: 2mL/min. | |
| **Detector wavelength** | 210 nm | |
| **Gradient** | Time (mins) | Composition A:B (v/v) |
| | 0.0 (Run start) | 50:50 |
| | 1.0 | 50:50 |
| | 12.0 (Run end) | 50:50 |
| **HPLC system 2** | Column Phenomenex Luna 5µ C-18 (2), 150 x 4.6 mm | |
| **Mobile phase** | Component A: 95:5 20mM pH7 KH₂PO₄:MeCN; | |
| | Component B: 25:75 20mM pH7 KH₂PO₄:MeCN; | |
| | Flow rate: 2mL/min. | |
| **Detector wavelength** | 228 nm | |
| **Gradient** | Time (mins) | Composition A:B (v/v) |
| | 0.0 (Run start) | 100:0 |
| | 8.0 | 0:100 |
| | 11.50 | 0:100 |
| | 11.55 | 100:0 |
| | 15.0 (Run end) | 100:0 |

LCMS retention times (RT) quoted were generated on a Hewlett Packard 1100 LC/MS using the following following method:
pH = 2.5 Phenomenex Luna 3µ C₁₈(2) 50x4.6mm column; mobile phase A = 0.1% formic acid in water; mobile phase B = 0.1% formic acid in MeCN; flow rate of 0.9mLmin⁻¹; column temperature 40°C.

| Gradient:- | Time (mins) | Composition A:B (v/v) |
|---|---|---|
| | 0.0 (Run start) | 95:5 |
| | 2.00 | 5:95 |
| | 3.00 | 5:95 |
| | 5.0 | 95:5 |
| | 5.5 | end |

pH = 5.8 Phenomenex Luna 5µ C₁₈(2) 100x4.6mm column; mobile phase A = 5mM NH₄OAc pH5.8; mobile phase B = 95:5 MeCN: 100mM NH₄OAc pH5.8; flow rate of 3.0mLmin⁻¹; column temperature 35°C.

| Gradient:- | Time (mins) | Composition A:B (v/v) |
|---|---|---|
| | 0.0 (Run start) | 95:5 |
| | 4.40 | 5:95 |
| | 5.30 | 5:95 |
| | 5.32 | 95:5.0 |
| | 6.50 | 95:5.0 |

Various methods including Karl-Fisher analysis, X-ray powder diffraction (XRPD), single-crystal X-ray, infra-red spectroscopy, differential scanning calorimetry (DSC) and dynamic vapour sorption studies are also used to characterise Examples 2, 2a, 3 and 4.

### Example 1 4-[2-Pyrrolidin-1-ylvinyl]nicotinonitrile

### Stage 1 - Condensation of pyrrolidine and dimethylformamide dimethylacetal

Dimethylformamide dimethylacetal (11.21g, 1.11 equivalents) was charged to pyrrolidine (10.22g, 1.70 equivalents) at ambient temperature. The mixture was heated to 83°C and stirred at this temperature for 1.5 hours, the reaction set for atmospheric distillation and distilled until an internal temperature of 118°C was achieved, then allowed to cool and the resultant mobile oil used directly in the next step.

### Stage 2 - Enamine formation

3-Cyano-4-methylpyridine (10.00g, 1.0 equivalent) was slurried in IPA (10ml), and the condensation product from *Stage 1* charged at ambient temperature, followed by an IPA rinse (10ml). The mixture was heated to reflux and maintained at reflux for 2 hours, when HPLC (system 1) analysis showed 98% conversion. The reaction was allowed to cool and stirred at ambient overnight, after which the solid was collected by filtration, slurried on the filter with IPA (16ml) then washed through with IPA (7ml). The solid was dried *in vacuo* to give the title compound, yield 14.76g, 87%. δH (CDCl₃) 8.49(1H, s), 8.23(1H, d J 5.9Hz), 7.56(1H, d, J 13.2Hz), 7.09(1H, d, J 5.9Hz), 5.21(1H, d, J 13.2Hz), 3.40(4H, m), 2.00(2H, t, J 6.6Hz). LCMS pH 5.8 (ES⁺) RT 2.92 minutes, 200 (M+H)⁺.

Amines other than pyrrolidine are also suitable, a table of which is given below. In a representative example, piperidine (1.19g, 1.65 equivalents) and *N,N-*dimethylformamide dimethylacetal (1.11g, 1.10 equivalents) were dissolved in IPA (2ml) and heated to reflux for 2 hours. At this time 3-cyano-4-methylpyridine (1.0g, 1.0 equivalents) was added and the reaction refluxed for a further 2 hours. LCMS pH 5.8 data for this and the other amines is given below:

| Amine | RT/min. | (M+H)⁺ |
|---|---|---|
| Piperidine | 3.23 | 214 |
| 2-Methylpiperidine | 3.50 | 228 |
| 3-Methylpiperidne | 3.58 | 228 |
| 4-Methylpiperidine | 3.59 | 228 |
| 3,5-Dimethylpiperidine (mix of *cis* and *trans)* | 3.80,3.94 | 242 |
| 2,6-Dimethylmorpholine | 2.97 | 244 |
| 2-Methylpyrrolidine | 3.25 | 214 |

### Example 2 2,7-Naphthyridin-1-ol hydrobromide

### Method A

Example 1, 668.6g (1 equiv) was slurried in AcOH, 835ml (1.25 volumes) and cooled with stirring to 10-15 °C. Trifluoroacetic acid, 1.84L (2.75 volumes) was charged, maintaining the temperature below 20 °C, during the addition. The resulting solution was added to a mixture of water, 135ml (0.2 volumes, 2.2 equivs) and HBr, 1.35L (45% w/w in AcOH, 2 volumes, 2.2 equivs) whilst maintaining the temperature below 45 °C. The mixture was stirred at 40-50 °C, until conversion was deemed complete by HPLC (system 1) (1.5-2h). The mixture was set-up for vacuum distillation, and the pressure reduced cautiously to achieve distillation (4.5-5 volumes collected, @ 110-70 mbar, 40-60 °C). Toluene (2 volumes) was charged and solvent distilled (2 - 2.5 volumes, @ 110-70 mbar, 40-60 °C) and the process repeated. Ethanol (3.5 volumes) was charged and a proportion of the reaction solvent removed by distillation (1.3-1.6 volumes, @ 110-70 mbar, 40-60 °C). The mixture was allowed to cool, stirred at ambient and the precipitate collected by filtration. The filter cake was slurried with ethanol (1.7 volumes), filtered and dried *in vacuo* at 40-50 °C, to give the *title compound* as an off white solid (745.9g, 97.9%). δH (DMSO) 9.47 (1H, s), 8.78 (1H, d, J 6.3Hz), 8.08 (1H, d, J 6.3Hz), 7.83 (1H, t, J 6.8Hz), 6.81 (1H, d, J 7.0Hz). LCMS pH2.5 (ES⁺) RT 0.53 minutes, 147 (M+H)⁺.

Karl-Fisher analysis 0.9% w/w water.

Infra-Red Spectroscopy (PE Spectrum, ATR sampling) is characterised by (but not restricted to) peaks at 1653, 1633, 1247, 1223, 813, 796 & 759 cm⁻¹.

Differential scanning calorimetry (DSC) (Mettler Toledo DSC12E, Aluminium pan, scan rate 10°C/min, 30-320°C) shows an endotherm with onset ca 265°.

### Method B

Similarly, 4-[2-piperidin-1-yl-vinyl]nicotinonitrile (18.79g) was cyclised as described in Method A to give crude *title compound,* 19.64g(98%), LCMS (pH 5.8) RT 1.38 minutes, (M+H)⁺ 147.

### Example 2a 2,7-Naphthyridin-1-ol hydrobromide hydrate

Karl-Fisher analysis 7.5% w/w water, equating to 1.0 moles water per mole of 2,7-naphthyridin-1-ol hydrobromide.

Infra-Red spectroscopy (PE Spectrum, ATR sampling) is characterised by (but not restricted to) peaks at ca 3394, 1653, 1633, 1253, 1229, 837 & 781 cm⁻¹. The broad band at 3394 cm⁻¹ is characteristic of inter-molecular, hydrogen-bonded -OH group.

DSC (Mettler Toledo DSC12E, Aluminium pan, scan rate 10°C/min, 30-320°C) shows a broad endotherm onset ca 65°C, followed by a similar endotherm with onset ca 265°C.

### Example 3 2,7-Naphthyridin-1-ol hydrochloride

4-[2-Pyrrolidin-1-ylvinyl]nicotinonitrile (example 1) (2.00g) was added, at room temperature, to SG1.18 HCl (10ml) over a period of 5 minutes. The solution was stirred at ambient temperature for 50 minutes then heated to 80°C for 20 minutes, when analysis by HPLC (system 1) indicated complete conversion. The reaction was allowed to cool then concentrated under reduced pressure, and azeotroped four times with ethanol. To this residue was added ethanol (4.5ml) and the resulting suspension stirred at ambient for 2 hours, after which time the solid was isolated by filtration, washed with ethanol (2x1ml) and dried *in vacuo* to give the title compound, yield 1.64g (90%) as a green solid. δH (MeOD) 9.52 (1H, s), 8.75 (1H, d, J 6.5Hz), 8.15 (1H, d, J 6.5Hz), 7.87 (1H, d, J 7.1Hz), 6.88 (1H, d, J 7.1Hz). LCMS pH2.5 (ES⁺) RT 0.53 minutes, 147 (M+H)⁺.

Karl Fischer analysis (isolated from dry ethanol) 0.93%w/w water.

X-Ray Powder Diffraction (XRPD) patterns (bulk material) were collected on a Siemens D5000 diffractometer using CuK_{α} radiation. Characterised by (but not restricted to) the following peaks: 9.4, 13.1, 14.0, 14.3,15.8,16.8,20.5, 23.4, 23.8, 25.0, 25.3, 26.7, 27.0, 27.5, 28.0, 28.3, 29.3, 30.8, 35.8, 36.5 degrees 2 theta.

Infra-red spectroscopy (PE Spectrum, ATR sampling) is characterised by (but not restricted to) peaks at 1678, 1611, 1462, 845, 806 & 798 cm⁻¹.

DSC (Mettler Toledo DSC12E, Aluminium pan, scan rate 10°C/min, 20-220°C) shows a single broad endotherm, onset ca 155°C and ending by 210°C.

Dynamic Vapour Sorption studies (Surface Measurement Systems DVS 1000) at 25°C show a very slow loss of ca 1.2 % w/w at 0% RH over eight hours. This loss is swiftly recovered on raising the RH above 10%, and there is a further slow gain of ca 1% w/w by 95% RH. The changes in weight with RH were reproducible across two RH cycles.

### Example 4 2,7-Naphthyridin-1-ol hydrochloride hydrate

### Method A

4-[2-Pyrrolidin-1-ylvinyl]nicotinonitrile (example 1) (10.01g) was added to SG1.18 HCl (50ml) over a period of 5 minutes at room temperature. The reaction was then heated to 80°C, analysis by HPLC (system 1) after 35 minutes indicating complete conversion. The reaction was cooled to 40°C and vacuum applied, and 2.5ml of distillate removed. To the reaction was then added propan-1-ol (40ml), vacuum re-applied and 30ml of distillate removed. A second portion of propan-1-ol was added, vacuum re-applied and 27ml of distillate removed. The reaction was allowed to cool to ambient, and after stirring for 15 minutes the solid was collected by filtration, slurried on the filter with propan-1-ol (15ml) and washed through with propan-1-ol (2x10ml). This solid was dried *in vacuo* to give the title compound as a pale yellow solid, yield 8.88g (88%). NMR data as above in Example 3.

Karl Fischer analysis (crystallised from aqueous n-propanol or i-propanol) 9.2%w/w water, 1.0 equivalents, hydrate.

Single crystal X-ray data: Crystals were grown from aqueous n-propanol (Instrument: Nonius Kappa CCD) C8 H9 Cl N2 02, M = 200.62, Monoclinic, Space Group C2/c, a 12.4267(3) Angstroms b 7.5329(2) Angstroms c 18.7840(7) Angstroms, alpha = 90.00 degrees, beta = 94.0230(10) degrees, gamma = 90.00 degrees, V = 1754.02 cubic Angstroms, T = 120 K, Z = 8, Density (calculated) = 1.519 g/cm³, λ = 0.71073 Angstroms. Final residuals for 124 parameters were R₁ {I>2sigma(I)}= 0.0411 and wR₂ = 0.0965.

The simulated x-ray powder pattern associated with this structure is characterised by (but not restricted to) the following peaks: 9.4, 13.8, 14.4, 14.7, 17.0, 17.7, 20.2, 23.6, 23.9, 25.3, 26.9, 27.1, 27.7, 27.9, 28.3, 28.8, 29.7, 31.0, 33.3, 35.5, 36.1, 36.8 degrees 2 theta (data simulated by Mercury 1.4.1 for λ = 1.54056 Angstroms).

An experimental x-ray powder diffraction pattern obtained from a bulk sample was found to be a match to the simulated one pattern above: Philips Xpert difractometer, using unmonochromated CuKα radiation, at laboratory ambient temperature, gave peaks at: 9.5, 13.7, 14.3, 14.6, 16.8, 17.6, 20.0, 23.3, 23.8, 25.1, 26.7, 27.0, 27.5, 27.7, 28.0, 28.6, 29.4, 30.8, 33.3, 35.1, 35.6, 36.6 degrees 2 theta.

Infra-red spectroscopy (PE Spectrum, ATR sampling) is characterised by (but not restricted to) peaks at ca 3383, 1678, 1635, 1462, 845, 812 & 783 cm⁻¹. The broad band at 3383 cm⁻¹, is characteristic of inter-molecular hydrogen-bonded -OH groups, and is consistent with the x-ray crystal structure for this material.

DSC (Mettler Toledo DSC12E, Aluminium pan, scan rate 10°C/min, 20-220°C) shows three events: an initial broad endotherm, onset ca 125°C, a sharp endotherm onset ca 165°C, and a final broad endotherm onset ca 190°C.

Dynamic Vapour Sorption studies (Surface Measurement Systems DVS 1000) at 25°C showed essentially no weight loss or gain over 2 cycles covering the range 0-95% RH, implying considerable stability.

### Method B

4-[2-Pyrrolidin-1-ylvinyl]nicotinonitrile (example 1) (20.00g) was added to SG1.18 HCl (60ml) at room temperature, and this addition rinsed in with a further charge of SG1.18 HCl (20ml). The resulting solution was heated to 80°C over 1 hour and maintained at this temperature for 20 minutes. It was then cooled to 40°C and propan-2-ol (100ml) added over thirty minutes. Vacuum was applied and 96ml of distillate removed. Another charge of propan-2-ol (100ml) was made, vacuum re-applied and 63ml of distillate removed. The mixture was then cooled to 20°C, stirred for 2.75 hours and the solid collected by filtration. The solid was washed through with 20ml 10% aqueous propan-2-ol, slurried on the sinter with 30ml 10% aqueous propan-2-ol and washed with a further 20ml of 10% aqueous propan-2-ol. The solid was dried *in vacuo* to yield the title compound as a yellow solid, 18.47g (92%), NMR data as above in example 3.

### Example 5 2,7-Naphthyridin-1-ol

### Method A

Example 1 (1.00g, 1 equivalent) was charged to a flask followed by 85wt% H₃PO₄ (10ml) and heated to 90°C. After 35 minutes sampled and analysed by LCMS pH5.8 RT 1.38min, (M+H)⁺ 147.

### Method B

Example 1 (1.0g) was suspended/dissolved in glacial acetic acid (3.88 ml) and water (90.5 µl, leq), then stirred at ambient temperature for the addition of 45% w/v HBr in AcOH (3.62 ml, 4.0 eq). The reaction was then heated at 40°C and sampled at intervals for analysis by hplc. After 4hrs 40 minutes the conversion was complete, and a 73% solution yield of the title compound was obtained.

### Example 6 1-Chloro-2,7-naphthyridine from the 2,7-Naphthyridin-1-ol hydrobromide

Phosphorus oxychloride, 1.645kg (2 vols) was added to Example 2, 0.50kg and the mixture stirred as DMF, 20g (0.1 mole eq) was charged from a dropping funnel. The mixture was then boiled under reflux for 3 hours. The top of the heat exchanger was vented through a caustic soda filled scrubber *via* an oil-filled gas bubbler to remove the HCl evolved. The preparation was cooled to 20-25°C and sampled for HPLC (system 2) completion check before proceeding.

Whilst the reaction was in progress, a second vessel was charged with 0.88 ammonia, 3.65kg (8.3 vols) and water, 4.15kg (8.3 vols). This mixture was subsequently cooled to -10°C and ethyl acetate, 4.5kg (5 vols) added. The contents of the reaction vessel were then quenched into the contents of the second vessel, keeping the temperature <15°C. Cooling was switched off at the end of the addition and the mixture allowed to stir then settle. Once the organic layer had been drummed up, the aqueous phase was extracted with more ethyl acetate, 4.5kg (5 vols) charged *via* the reaction vessel and connecting line. After the second stir-out and separation, a third ethyl acetate wash, 1.35kg (2 vols) was charged to the reaction vessel and cooled back to 0-5°C. Aqueous liquors were then added slowly to the reaction vessel keeping the internal temperature <15°C until it was full. After a stir-out the mixture was transferred to the work-up vessel and the remaining aqueous liquors and ethyl acetate, 2.25kg (3 vols) employed as line washes. The whole was stirred out again in the work-up vessel prior to the third separation. The aqueous liquors were extracted for the fourth time with ethyl acetate, 4.5kg (5 volumes) charged to the work-up vessel *via* the reaction vessel. Following the fourth separation, the combined ethyl acetate solutions were filtered, back-washed with water (1 volume), and then dried over magnesium sulphate (0.25 wt eqs). The suspension was filtered and the inorganic cake washed with further ethyl acetate, 0.45kg (2 x 1 volume). Removal of the solvent *in vacuo* furnished the crude product that was sufficiently pure to be used without purification downstream, after drying *in vacuo* at 35-40°C to give the *title compound* as an off white solid (609g, 95.1%). NB mixture of 1-chloro-2,7-naphthyridine and 1-bromo-2,7-naphthyridine in a ratio of approximately 90: 10 Cl:Br as shown by HPLC (system 2). Data reported for the 1-Chloro-2,7-naphthyridine. δH (CDCl₃) 9.76 (1H, s), 8.83 (1H, d, J 5.7 Hz), 8.48 (1H, d, J 5:7 Hz), 7.67 (1H, d, J 5.7 Hz), 7.63 (1H, d, J 5.7 Hz). LCMS pH2.5 (ES⁺) RT 2.10 minutes, 165.2 (M+H)⁺. MP = 117.6°C.

### Example 7 1-Chloro-2,7-naphthyridine from the 2,7-Naphthyridin-1-ol hydrochloride hydrate

### Method A

Phosphorus oxychloride 108ml, 170g (2 vols) was added to Example 4, 60g (containing ~9% water by weight). The mixture was then boiled under reflux for 3.5 hours. The top of the heat exchanger was vented through a caustic soda filled scrubber *via* an oil-filled gas bubbler to remove the HCl evolved. The preparation was cooled to 20-25°C and sampled for a HPLC (system 2) which showed the reaction to have gone to completion. To the reaction mixture was added 240ml of DCM, to help wash out the vessel. The reactor was then set to 5-10°C and the above reaction mixture slowly and carefully added to a mixture of 0.88 ammonia (8 vols) 480ml and water (8 vols) 480ml, keeping the temperature below 15°C. Once the organic layer had been separated, the aqueous phase was extracted with more DCM, 240ml which was also used to wash out the reaction vessel. The combined organic layers were then back-washed with water 180ml. Removal of the solvent *in vacuo* furnished the crude product that was sufficiently pure to be used without purification downstream, after drying *in vacuo* at 35-40°C to give the *title compound* as an off white solid (47.5g, 96.7%). δH (CDCl₃) 9.76 (1H, s), 8.83 (1H, d, J 5.7 Hz), 8.48 (1H, d, J 5.7 Hz), 7.67 (1H, d, J 5.7 Hz), 7.63 (1H, d, J 5.7 Hz). LCMS pH2.5 (ES⁺) RT 2.10 minutes, 165.2 (M+H)⁺. MP = 118.1-119.8°C.

### Method B

Phosphorus oxychloride 1500ml, 2469.5g (2 vols) was added to Example 4, 750g (containing ~9% water by weight). The mixture was then boiled under reflux for 2 hours. The top of the heat exchanger was vented through a caustic soda filled scrubber *viα* an oil-filled gas bubbler to remove the HCl evolved. The preparation was cooled to 20-25°C and sampled for a HPLC (system 2) which showed the reaction to have gone to completion. To the reaction mixture was added 4500ml of DCM, to help wash out the vessel. The reactor was then set to 5-10°C and the above reaction mixture slowly and carefully added to a mixture of 0.88 ammonia (8 vols) 6375ml and water (8 vols) 6375ml, keeping the temperature below 15°C. Once the organic layer had been separated, the aqueous phase was extracted with more DCM, 2 x 1500m which was also used to wash out the reaction vessel. The combined organic layers were then back-washed with water 3750ml. The resulting DCM solution was then distilled at atmospheric pressure, with the removal of ~6600ml of solvent. To the distillation vessel, was added acetonitrile 900ml and the distillation continued, with the removal of a further 900ml of distillate. This process was repeated with another addition of acetonitrile 900ml and removal of 900ml of distillate. To the resulting residue, was added water 3000ml over a 30minute period, keeping the internal temperature above 50°C. The slurry was then cooled gradually to 5°C held at this point for an hour and the solid collected by filtration, washing with water 1500ml. After drying *in vacuo* at 35-40°C to give the *title compound* as an off white solid (526.5g, 85.5%). HPLC >99.8% purity. δH (CDCl₃) 9.76 (1H, s), 8.83 (1H, d, J 5.7 Hz), 8.48 (1H, d, J 5.7 Hz), 7.67 (1H, d, J 5.7 Hz), 7.63 (1H, d, J 5.7 Hz). LCMS pH2.5 (ES⁺) RT 2.10 minutes, 165.2 (M+H)⁺. MP = 118.1-119.8°C.

### Example 8 1-Chloro-2,7-naphthyridine from the 2,7-Naphthyridin-1-ol (Alternative chlorination procedure)

A mixture of 2,7-naphthyridin-1-ol (Example 5) 1.0g (6.85mmol), tetrabutylammonium chloride 2.21g (7.95mmol) and phosphorus pentoxide 2.31g (0.016mole) in 15ml of toluene were stirred in a round bottomed flask. The mixture was then boiled under reflux for 4.5 hours. The preparation was cooled to 20-25°C and sampled for HPLC (system 2) which showed the reaction to have gone to approximately 20% completion. The toluene solvent was removed from the solid mass and removal of the solvent *in vacuo* furnished the crude product that was sufficiently pure to be used without purification downstream, after drying *in vacuo* at 35-40°C to give the *title compound* as a white solid (200mg, 17.8%). δH (CDCl₃) 9.76 (1H, s), 8.83 (1H, d, J 5.7 Hz), 8.48 (1H, d, J 5.7 Hz), 7.67 (1H, d, J 5.7 Hz), 7.63 (1H, d, J 5.7 Hz). LCMS pH2.5 (ES⁺) RT 2.10 minutes, 165.2 (M+H)⁺. MP = 118.1-119.8°C.

### Example 9 1-Bromo-2,7-naphthyridine directly from the enamine Method A

Example 1 (100g) was dissolved in 15-20 volumes of dichloromethane (1.5-2.0L) and cooled down to -5°C. To this solution was added gaseous hydrogen bromide. During the course of the reaction the mixture was allowed to warm to 20°C. The conversion was monitored by HPLC (system 2) and was complete after approximately 5 hours. The reaction mixture was then poured into saturated NaHCO₃, and the layers separated with the aqueous one being further extracted with dichloromethane. The combined organic layers were then dried (MgSO₄) and then evaporated to dryness. The crude bromonaphthyridine 97.7g (93%) was purified by recrystallisation from methanol/water, after drying *in vacuo* at 35-40°C to give the *title compound* as a light brown solid (59.9g, 57%). δH (CDCl₃) 9.70 (1H, s), 8.82 (1H, d, J 6.0 Hz), 8.46 (1H, d, J 5.7 Hz), 7.63 (2H, t, J 5.3 Hz). LCMS pH2.5 (ES⁺) RT 2.22 minutes, 211.1 (M+H)⁺.

### Method B

Example 1 (1.0g) was suspended/dissolved in glacial acetic acid (3.88 ml) and stirred at ambient temperature for the addition of 45% w/v HBr in AcOH (3.62 ml, 4.0 eq). The reaction was then heated at 40°C and sampled at intervals for analysis by hplc. After 30 minutes the conversion of the enamine was complete, and a 68% solution yield of the title compound was obtained.

### Example 10 1-Cbloro-2,7-napbthyridine directly from the enamine

Example 1 (1.0g) was suspended/dissolved in glacial acetic acid (3.65 ml) and stirred at ambient temperature for the addition of HCl in AcOH (~5.3 M, 3.85 ml, 4.0 eq). The reaction was then heated at 40°C and sampled at intervals for analysis by hplc. At 1hr 50 mins the conversion of the enamine was incomplete, but a 13% solution yield of the title compound was obtained.

### Example 11 2,6-Dichloro-4-(2-pyrrolidin-1-ylvinyl)nicotinonitrile

To a flask was charged dimethylformamide dimethylacetal (7.18g, 1.1 equivalents) and pyrrolidine (6.39g, 1.7 equivalents) and the mixture heated to 95°C for two hours. At this time the reaction was distilled until the internal temperature reached 114°C and the resulting oil was stored under nitrogen. To a separate flask 2,6-dichloronicotinonitrile (10.05g, 1.00 equivalents) and propan-2-ol (10ml) was added, and to this mixture was added the oil from the first step, along with a further 10ml propan-2-ol. This caused the internal temperature to rise to 45°C and a red precipitate to form. The reaction was stirred at ambient temperature for 2 hours, when the solid was isolated by filtration. The filter cake was slurried with propan-2-ol (10ml), washed through with propan-2-ol (10ml) and dried *in vacuo* to yield the title compound, 4.94g (34%). LCMS (pH 5.8) RT 4.17 minutes, [M+H]⁺ 268/270.

### Example 12 6,8-Dichloro-[2,7]naphthyridin-1-ol

To a flask was added c.HCl (8ml), to which was added Example 11 (1.00g) and the addition washed in with c.HCl (2ml). The reaction was heated to 60°C for 90 minutes and then allowed to cool to room temperature. The solid was isolated by filtration, washed through with water (2ml) and dried *in vacuo* to give the title compound, 0.25g (31%). LCMS (pH 5.8) RT 2.34 minutes, [M+H]⁺215/217.

### Example 13 [2,7]naphthyridin-1-ol

To a flask was charged Example 12 (0.20g, 1.00 equivalents), 5% Pd/C (0.02g, 10wt%), sodium acetate (0.31g, 4.1 equivalents) and methanol (10ml). This was then stirred at ambient temperature under atmospheric pressure of hydrogen for 22¼ hours. The reaction was filtered through Celite^{®} and concentrated under reduced pressure. The residue was partitioned between DCM (10ml) and water (5ml) and the aqueous layer extracted with DCM (2x5ml). The organics were combined, dried with sodium sulfate, filtered and concentrated under reduced pressure to give the title compound, 0.09g (64%). LCMS (pH 5.8), RT 1.38 minutes, [M+H]⁺ 147.

## Claims

1. A process for the preparation of a compound of formula (I): 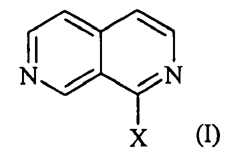 wherein
X is Cl or Br;
which process comprises the following steps:
(i) reaction of a 3-cyano-4-methylpyridine derivative of formula (A): 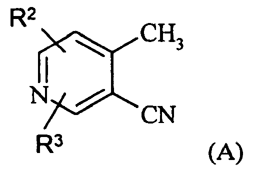 with a compound of formula (II): 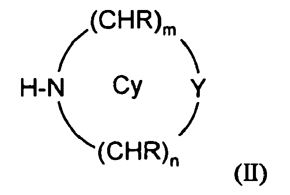 in the presence of an *N,N*-dimethylformamide diC₁₋₆alkylacetal;
to give an enamine derivative of formula (III): 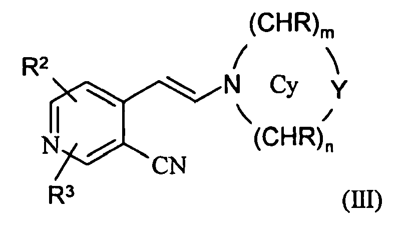 wherein R² and R³ independently represent hydrogen, Cl or Br; and Cy represents a N-linked 4 to 6 membered heterocyclic ring in which m is 1 or 2; n is 1 or 2; Y represents -CHR-, -NR¹-, -O- or -S(O)ₓ-; x is zero, 1 or 2; each R independently represents hydrogen or C₁₋₆alkyl; and R¹ represents hydrogen or C₁₋₆alkyl;
(ii) cyclisation of the enamine of formula (III), followed, as necessary, by removal of all Cl or Br atoms, to obtain the compound of formula (IV): 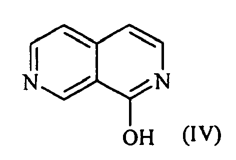
(iii) reaction of the compound of formula (IV) with a halogenating agent, to obtain a compound of formula (I).

2. A process according to Claim 1 wherein Cy represents pyrrolidin-1-yl.

3. A process according to Claim 1 or 2 wherein, in step (ii), the cyclisation is performed using HBr, HCl or H₃PO₄.

4. A process according to any one of Claims 1 to 3 wherein, in step (iii), the halogenating agent is phosphorus oxychloride, phosphorus oxybromide or is generated from tetra-n-butylammonium chloride and phosphorus pentoxide.

5. A process according to any one of Claims 1 to 4 wherein the hydrobromide or hydrochloride salt of the compound of formula (IV) is used in step (iii) of the process.

6. A process according to Claim 5 wherein the hydrate of the hydrobromide or hydrochloride salt of the compound of formula (IV) is used in step (iii) of the process.

7. A process according to Claim 5 or 6 wherein the compound of formula (IV) is 2,7-naphthyridin-1-ol hydrochloride hydrate.

8. A compound of formula (III): 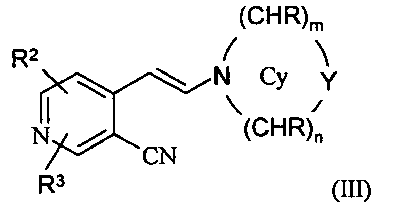 wherein R² and R³ independently represent hydrogen, Cl or Br; and Cy represents a N-linked 4 to 6 membered heterocyclic ring in which m is 1 or 2; n is 1 or 2; Y represents -CHR-, -NR¹-, -O- or -S(O)ₓ-; x is zero, 1 or 2; each R independently represents hydrogen or C₁₋₆alkyl; and R¹ represents hydrogen or C₁₋₆alkyl.

9. A process for the preparation of a compound of formula (I), as defined in Claim 1, which comprises reacting a compound of formula (III), as defined in Claim 1 with HBr or HCl.

## Patentansprüche

1. Verfahren zur Herstellung einer Verbindung der Formel (I): 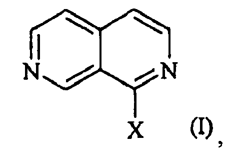 worin
X Cl oder Br ist;
wobei das Verfahren die folgenden Schritte umfasst:
(i) Umsetzen eines 3-Cyano-4-methylpyridinderivats der Formel (A): 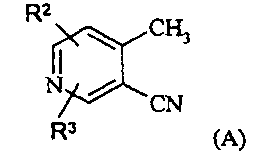 mit einer Verbindung der Formel (II): 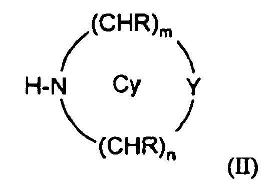 in Gegenwart eines *N*,*N*-Dimethylforrnamid-di-C₁₋₆-alkylacetals;
unter Erhalt eines Enaminderivats der Formel (III): 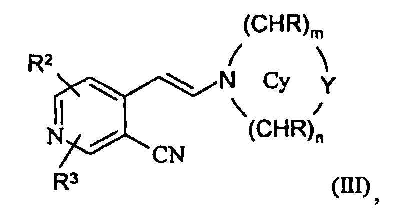 worin R² und R³ unabhängig voneinander Wasserstoff, Cl oder Br darstellen und Cy einen N-verknüpften 4- bis 6gliedrigen heterocyclischen Ring darstellt, bei dem m 1 oder 2 ist; n 1 oder 2 ist; Y -CHR-, -NR¹-, -O- oder -S(O)ₓ- darstellt; x Null, 1 oder 2 ist; jeder R unabhängig Wasserstoff oder C₁₋₆-Alkyl darstellt und R¹ Wasserstoff oder C₁₋₆-Alkyl darstellt;
(ii) Cyclisieren des Enamins der Formel (III), gefolgt, wenn notwendig, von der Entfernung aller Cl- oder Br-Atome, unter Erhalt der Verbindung der Formel (IV): 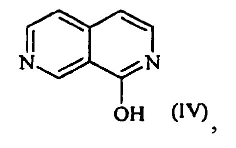
(iii) Umsetzen der Verbindung der Formel (IV) mit einem Halogenierungsmittel unter Erhalt einer Verbindung der Formel (I).

2. Verfahren nach Anspruch 1, wobei Cy Pyrrolidin-1-yl darstellt.

3. Verfahren nach Anspruch 1 oder 2, wobei in Schritt (ii) die Cyclisierung unter Verwendung von HBr, HCl oder H₃PO₄ durchgeführt wird.

4. Verfahren nach einem der Ansprüche 1 bis 3, wobei in Schritt (iii) das Halogenierungsmittel Phosphor(V)-oxidchlorid, Phosphor(V)-oxidbromid ist oder aus Tetra-*n*-butylammoniumchlorid und Phosphorpentoxid generiert wird.

5. Verfahren nach einem der Ansprüche 1 bis 4, wobei das Hydrobromid- oder Hydrochloridsalz der Verbindung der Formel (IV) in Schritt (iii) des Verfahrens verwendet wird.

6. Verfahren nach Anspruch 5, wobei das Hydrat des Hydrobromid- oder Hydrochloridsalzes der Verbindung der Formel (IV) in Schritt (iii) des Verfahrens verwendet wird.

7. Verfahren nach Anspruch 5 oder 6, wobei die Verbindung der Formel (IV) 2,7-Naphthyridin-1-ol-hydrochloridhydrat ist.

8. Verbindung der Formel (III): 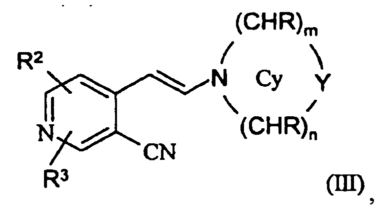 worin R² und R³ unabhängig voneinander Wasserstoff, Cl oder Br darstellen und Cy einen N-verknüpften 4- bis 6 gliedrigen heterocyclischen Ring darstellt, bei dem m 1 oder 2 ist; n 1 oder 2 ist; Y -CHR-, -NR¹-, -O- oder -S(O)ₓ- darstellt; x Null, 1 oder 2 ist; jeder R unabhängig Wasserstoff oder C₁₋₆-Alkyl darstellt und R¹ Wasserstoff oder C₁₋₆-Alkyl darstellt.

9. Verfahren zur Herstellung einer Verbindung der Formel (I), wie in Anspruch 1 definiert, welches das Umsetzen einer Verbindung der Formel (III), wie in Anspruch 1 definiert, mit HBr oder HCl umfasst.

## Revendications

1. Procédé de préparation d'un composé de formule (I) : 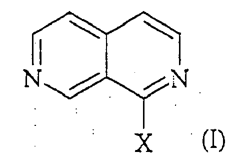 dans laquelle
X est Cl ou Br ;
ce procédé comprenant les stades suivants :
(i) réaction d'un dérivé de 3-cyano-4-méthylpyridine de formule (A) : 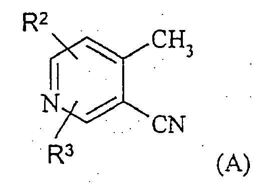 sur un composé de formule (II) : 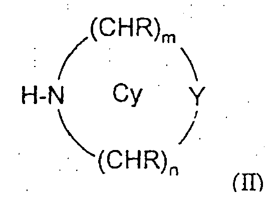 en la présence d'un N,N-diméthylformamide dialcoylacétal ayant de 1 à 6 atomes de carbone dans la partie alcoyle ;
pour donner un dérivé d'énamine de formule (III) : 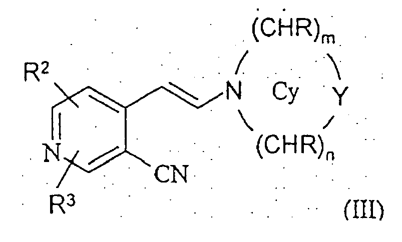 dans laquelle R² et R³ représentent indépendamment hydrogène , Cl ou Br ; et Cy représente un cycle hétérocyclique à liaison N ayant de 4 à 6 chaînons, dans lequel m est 1 ou 2 ; n est 1 ou 2 ; Y représente -CHR-, -NR¹-, -0- ou -S(O)ₓ- ; x est zéro, 1 ou 2 ; chaque R représente indépendamment hydrogène ou alcoyle ayant de 1 à 6 atomes de carbone ; et R¹ représente hydrogène ou alcoyle ayant de 1 à 6 atomes de carbone ;
(ii) cyclisation de l'énamine de formule (III), suivie, si nécessaire, de l'élimination de tous les atomes de Cl ou de Br pour obtenir le composé de formule (IV) : 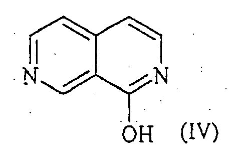
(iii) réaction du composé de formule (IV) sur un agent d'halogénation pour obtenir un composé de formule (I).

2. Procédé suivant la revendication 1, dans lequel Cy représente pyrrolidine-1-yl.

3. Procédé suivant la revendication 1 ou 2, dans lequel, dans le stade (ii), on effectue la cyclisation en utilisant HBr, HCl ou H₃PO₄.

4. Procédé suivant l'une quelconque des revendications 1 à 3, dans lequel, dans le stade (iii), l'agent d'halogénation est de l'oxychlorure de phosphore, de l'oxybromure de phosphore ou est produit à partir de chlorure de tétra-*n*-butylammonium et de pentoxyde de phosphore.

5. Procédé suivant l'une quelconque des revendications 1 à 3, dans lequel on utilise le bromhydrate ou le chlorhydrate du composé de formule (IV) dans le stade (iii) du procédé.

6. Procédé suivant la revendication 5, dans lequel on utilise l'hydrate du bromhydrate ou du chlorhydrate du composé de formule (IV) dans le stade (iii) du procédé.

7. Procédé suivant la revendication 5 ou 6, dans lequel le composé de formule (IV) est l'hydrate du chlorhydrate de 2,7-naphtyridine-1-ol.

8. Composé de formule (II) : 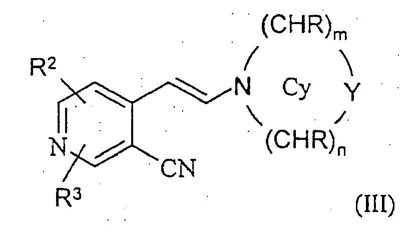 dans laquelle R² et R³ représentent indépendamment hydrogène , Cl ou Br ; et Cy représente un cycle hétérocyclique à liaison N ayant de 4 à 6 chaînons, dans lequel m est 1 ou 2 ; n est 1 ou 2 ; Y représente -CHR-, -NR¹⁻, -O- ou -S(O)ₓ- ; x est zéro, 1 ou 2 ; chaque R représente indépendamment hydrogène ou alcoyle ayant de 1 à 6 atomes de carbone ; et R¹ représente hydrogène ou alcoyle ayant de 1 à 6 atomes de carbone.

9. Procédé de préparation d'un composé de formule (I) tel que défini à la revendication 1, dans lequel on fait réagir un composé de formule (III) tel que défini à la revendication 1 sur HBr ou HCl.
